# FASCICULE DE BREVET EUROPEEN

(11) **EP 1 397 161 B1**
(45) Date de publication et mention de la délivrance du brevet: **30.04.2008**
(21) Numéro de dépôt: 02704883.4
(22) Date de dépôt: 22.02.2002
(51) Int. Cl.: A61K 47/48, A61P 23/00

(54) **COMPOSES CONSTITUES D'UNE MOLECULE ANALGESIQUE LIEE A UN VECTEUR**
VERBINDUNGEN DIE EIN AN EINEN VEKTOR GEBUNDENES ANALGETISCHES MOLEKÜL ENTHALTEN
COMPOUNDS COMPRISING AN ANALGESIC MOLECULE LINKED TO A VECTOR

(30) Priorité: 23.02.2001 FR 0102504
(43) Date de publication de la demande: 17.03.2004
(73) Titulaire: SYNT:EM, 30000 Nîmes (FR)
(72) Inventeur: TEMSAMANI, Jamal, F-30900 Nîmes (FR); REES, Anthony, R., F-30190 Saint-Chaptes (FR); CLAIR, Philippe, F-30900 Nîmes (FR)
(74) Mandataire: Breese, Pierre
(86) Numéro de dépôt international: PCT/FR2002/000667
(87) Numéro de publication internationale: WO 2002/067994

(56) Documents cités:
- WO-A-00/32236
- WO-A-00/32237
- WO-A-99/07728
- ROUSSELLE C ET AL: "NEW ADVANCES IN THE TRANSPORT OF DOXORUBICIN THROUGH THE BLOOD-BRAIN BARRIER BY A PEPTIDE VECTOR-MEDIATED STRATEGY" MOLECULAR PHARMACOLOGY, vol. 57, no. 4, avril 2000 (2000-04), pages 679-686, XP001004535 ISSN: 0026-895X
- DEROSSI D ET AL: "TROJAN PEPTIDES: THE PENETRATIN SYSTEM FOR INTRACELLULAR DELIVERY" TRENDS IN CELL BIOLOGY, ELSEVIER SCIENCE LTD, XX, vol. 8, février 1998 (1998-02), pages 84-87, XP002940006 ISSN: 0962-8924
- ROUSSELLE CHRISTOPHE ET AL: "Enhanced delivery of doxorubicin into the brain via a peptide-vector-mediated strategy: Saturation kinetics and specificity." JOURNAL OF PHARMACOLOGY AND EXPERIMENTAL THERAPEUTICS, vol. 296, no. 1, janvier 2001 (2001-01), pages 124-131, XP001041627 ISSN: 0022-3565
- ROQUES BP: "Peptidomimetics as receptors agonists or peptidase inhibitors: a structural approach in the field of enkephalins, ANP and CCK." BIOPOLYMERS, APR 1992, VOL. 32, NO. 4, PAGE(S) 407-10, XP001041605
- MIGNAT CHRISTIAN ET AL: "Plasma and Cerebrospinal Fluid Concentrations of Morphine and Morphine Glucuronides in Rabbits Receiving Single and Repeated doses of Morphine." JOURNAL OF PHARMACY AND PHARMACOLOGY, vol. 47, no. 2, 1995, pages 171-175, XP001041620 ISSN: 0022-3573
- NEGRI L ET AL: "Production of antinociception by peripheral administration of [Lys7]dermorphin, a naturally occurring peptide with high affinity for mu-opioid receptors." BRITISH JOURNAL OF PHARMACOLOGY, JAN 1995, VOL. 114, NO. 1, PAGE(S) 57-66, XP001041563
- LOETSCH JOERN ET AL: "Pharmacokinetics of morphine and its glucuronides after intravenous infusion of morphine and morphine-6-glucuronide in healthy volunteers." CLINICAL PHARMACOLOGY & THERAPEUTICS, vol. 60, no. 3, 1996, pages 316-325, XP001041619 ISSN: 0009-9236

### SEQUENCE LISTING

<110> SYNT:EM
<120> COMPOSES CONSTITUES D'UNE MOLECULE ANALGESIQUE LIEE A UN VECTEUR CAPABLE DE VECTORISER LADITE MOLECULE A TRAVERS LA BARRIERE HEMATO-ENCEPHALIQUE ET COMPOSITIONS PHARMACEUTIQUES LES CONTENANT.
<130> 12054PCT
<140> PCT/FR02/XXXXXX
   <141> 2002-02-22
<150> FR 01/02504
   <151> 2001-02-23
<160> 1
<170> PatentIn version 3.1
<210> 1
   <211> 10
   <212> PRT
   <213> unidentified
<220>
   <221> PEPTIDE
   <222> (1)..(10)
   <223> Peptide SynB3
<400> 1
   Arg Arg Leu Ser Tyr Ser Arg Arg Arg Phe
   1               5                   10

## Description

La présente invention se rapporte à des composés constitués d'une molécule analgésique vectorisée par sa liaison à un vecteur de manière à ce que ladite molécule analgésique traverse la barrière hémato-encéphalique, ainsi qu'à l'utilisation desdits composés pour la préparation de médicaments utiles pour le traitement de la douleur.

La morphine constitue un des composés les plus utilisés dans le traitement des douleurs de moyenne et de grande intensité. Malheureusement, le traitement avec la morphine est souvent accompagné par des effets indésirables tels que : euphorie ou somnolence, dépression respiratoire, inhibition du transit intestinal, nausées, vomissements, et surtout syndrome de dépendance et induction de tolérance (Cherny et al, 1996). Après son administration chez l'animal ou le patient, la morphine subit un effet important de premier passage hépatique, ce qui a pour conséquence une biodisponibilité faible et variable selon la voie d'administration. La morphine subit principalement une glucuronidation énantiosélective catalysée par l'enzyme UDP-glucuronyltransférase (UGT) et le foie apparaît comme le site principal de sa biotransformation. Un des principaux dérivés de la morphine est son métabolite, la morphine 6-glucuronide (M6G).

Ce métabolite possède lui aussi une activité analgésique. Des études de liaison ligand-récepteur aux opiacés réalisées *in vitro* ont montré que la M6G se liait aux récepteurs opioïdes et qu'elle était 3 à 5 fois moins affine pour les récepteurs µ que la morphine (Christensen & Jorgensen 1987 ; Frances et al, 1992). Les récepteurs µ sont de deux types : des récepteurs µl de très haute affinité et faible capacité, et des récepteurs µ2 de base affinité et forte capacité (Pasternak & Wood, 1986). La liaison aux récepteurs µl entraîne une réaction analgésique de type supraspinal et la diminution du turnover de l'acétylcholine, tandis que la liaison aux récepteurs µ2 entraîne une réaction analgésique de type spinal et est responsable de la dépression respiratoire, de l'inhibition du transit intestinal et de plusieurs signes associés à la dépendance.

La morphine et son métabolite actif, la M6G, doivent franchir la barrière hémato-encéphalique (BHE) avant de se distribuer dans le cerveau, lieu d'action principal de leurs effets analgésiques. La morphine est une base dont le pKa se situe entre 8 et 9, donc faiblement ionisée au pH sanguin, et dont la pénétration cérébrale a été décrite comme une simple diffusion. Son métabolite, la M6G, est un acide (pKa entre 2 et 3), fortement ionisé au pH sanguin. A cause de son hydrophilie, la pénétration de la M6G est très limitée dans le cerveau.

Il a été démontré que l'administration intracérébroventriculaire, à dose égale, de la morphine et de la M6G produit un effet analgésique qui est 50 à 100 fois plus important pour la M6G que pour la morphine (Pasternak et al 1987 ; Frances et al, 1982 ; Stain et al 1995). Par contre, par injection systémique, les deux molécules ont à peu près la même activité analgésique. Ces résultats indiquent clairement que la pénétration de la M6G dans le cerveau est très limitée par rapport à celle de la morphine.

La barrière hémato-encéphalique est constituée par des cellules endothéliales qui font obstacle, de diverses manières, aux molécules qui tentent de les franchir. Elles constituent une barrière physique représentée par les jonctions étanches qui se lient entre elles et empêchent tout passage par la voie paracellulaire et ce, d'autant plus que l'activité d'endocytose y est faible. Tout ceci limite fortement le passage des molécules du plasma vers l'espace extracellulaire cérébral.

Ainsi, dans le cadre de ses travaux de recherche, la Demanderesse a mis en évidence que des vecteurs, comme les peptides linéaires dérivés de peptides naturels tels que la Protégrine et la Tachyplésine transportent des molécules actives à travers la BHE. La Protégrine et la Tachyplésine sont des peptides naturels dont la structure est de type épingle à cheveux maintenue par des ponts disulfures. Ces ponts jouent un rôle important dans l'activité cytolytique observée sur des cellules humaines. La réduction irréversible de ces ponts permet d'obtenir des peptides linéaires, non cytotoxiques, ayant la capacité de traverser rapidement les membranes des cellules de mammifères par un mécanisme passif ne faisant pas appel à un récepteur membranaire.

Les travaux et résultats concernant ces peptides linéaires et leur utilisation comme vecteurs de molécules actives à travers la barrière hémato-encéphalique ont été décrits dans les demandes de brevet français N° 98/15074 déposée le 30 Novembre 1998 et N° 99/02938 déposée le 26 Novembre 1999 par la Demanderesse.

La demande de brevet PCT WO 0032236 montre une augmentation de l'activité analgésique de la dalargine vectorisée à l'aide de SynB1 et teste le conjugué de SynB3 avec la doxorubicine.

L'article de Rousselle C. et al. (« New advances in the transport of doxorubicin through the Blood-Brain-Barrier by a peptide vector-mediated stragegy », Molecular Pharmacology, vol. 57, no. 4, avril 2000 (2000-04), pages 679-686, ISSN : 0026-895X) décrit que le conjugué doxorucine avec SynB1 passe la barrière hématoencéphalique.

L'article de Derossi D et al. (« Trojan Peptides : The penetratin system for intraoccular delivery », Trends en Cell Biology, vol. 8, février 1998 (1998-02), pages 84-87, ISSN : 0962-8924) décrit d'autres peptides.

La demande de brevet PCT WO 9907728 décrit des conjugués avec des peptides antibiotiques, dnt un peptide désigné SM2195 correspondant à SynB3.

La demande de brevet PCT WO 00 32237 décrit le même peptide couplé à la doxorubicine.

L'article de Rousselle Christophe et al. (Enhanced delivery of doxorubin into the brai via a peptide-vector-mediated strategy : Saturation kinetics and specificity » Journal of Pharmacology and Experimental Therapeutics, vol. 296, no. 1, janvier 2001 (20001-01), pages 124-131, ISSN : 0022-3565) décrit le transport augmenté du conjugué SynB3 et de la doxorubicine à travers la barrière hémato-méningée.

La présente invention vise à fournir un médicament analgésique présentant moins d'effets secondaires de la morphine. Ce but est atteint selon l'invention grâce à un conjugué de la morphine-6-glucuronide, métabolite de la morphine, avec le peptide SynB3, qui est un peptide vecteur linéaire dérivé de peptide antibiotique.

De manière préférentielle, le vecteur capable de vectoriser ladite molécule analgésique à travers la barrière hémato-encéphalique est un peptide linéaire dérivé de la famille de la Protégrine ou de la Tachyplésine.

On entend par peptide dérivé de la famille de la Protégrine tout peptide qui répond à la formule I suivante :

BXXBXXXXBBBXXXXXXB (I)

et par peptide dérivé de la famille de la Tachyplésine tout peptide qui répond à la formule II suivante :

BXXXBXXXBXXXXBBXB (II),

dans lesquelles :
- les groupes B, identiques ou différents, représentent un résidu d'acide aminé dont la chaîne latérale porte un groupement basique, et
- les groupes X, identiques ou différents, représentent un résidu d'acide aminé aliphatique ou aromatique
ou lesdits peptides de formules (I) ou (II), sous forme rétro, constitués d'acides aminés de configuration D et/ou L,
ou un fragment de ceux-ci constitué d'une séquence d'au moins 5 et de préférence d'au moins 7 acides aminés successifs des peptides de formules (I) ou (II).

A titre d'exemple de dérivés de la morphine, on peut citer ceux qui ont une activité analgésique mais qui ,en tant que tels, ne traversent pas la barrière hémato-encéphalique, comme les métabolites de la morphine, et notamment la M6G.

Préférentiellement, la molécule analgésique utilisée dans le cadre de la présente invention est la M6G.

Les positions de liaison pour la molécule analgésique peuvent être au niveau des extrémités N-terminale ou C-terminale ou bien au niveau des chaînes latérales du peptide vecteur.

Des groupements fonctionnels tels que -OH, - SH, -COOH, -NH₂ peuvent être naturellement présents ou peuvent être introduits, soit sur le vecteur, soit sur la molécule analgésique, soit sur les deux.

La liaison entre le vecteur et la molécule analgésique peut être réalisée par tout moyen de liaison acceptable compte tenu de la nature chimique et de l'encombrement tant du vecteur comme de la molécule analgésique. Les liaisons peuvent être covalentes, hydrophobes ou ioniques, clivables ou non-clivables dans les milieux physiologiques ou à l'intérieur des cellules.

Cette liaison entre le vecteur et la molécule analgésique peut être effectuée de manière directe ou indirecte.

Lorsque la liaison est effectuée de manière indirecte, on peut avantageusement utiliser un agent de liaison. A titre d'exemple non limitatif, d'agents de liaison utilisables dans le cadre de l'invention, on peut citer des agents bi- ou multifonctionnels contenant des groupements alkyle, aryle, aralkyle ou peptidique, des esters, aldéhydes ou acides d'alkyle, aryle ou aralkyle, des groupements anhydrides, sulfhydriles, ou carboxyles tels que les dérivés de l'acide maleymil benzoïque, de l'acide maleymil propionique et des dérivés succynimidyle, des groupes dérivés du bromure ou chlorure de cyanogène, carbonyldiimidazole, des esters de succinimide ou des halogénures sulphoniques.

Avantageusement, on utilise des liaisons impliquant au moins un pont disulfure, lesquelles se caractérisent par leur stabilité dans le plasma après injection du composé, puis une fois que les composés de l'invention ont traversé la barrière hémato-encéphalique, ledit pont disulfure est réduit en libérant la molécule analgésique active. La liaison peut être effectuée en n'importe quel site du vecteur.

le composé analgésique vectorisé est constitué par un dérivé de la morphine couplé par sa position 6 au vecteur.

Tout préférentiellement, lorsque la molécule analgésique est la morphine, le vecteur est fixé au niveau de la position 6 de ladite molécule de morphine.

Tout préférentiellement encore, lorsque la molécule analgésique est la morphine-6-glucuronide, le vecteur est fixé au niveau de l'acide carboxylique du résidu glucuronide de ladite molécule de morphine-6-glucuronide.

La présente invention a aussi pour objet l'utilisation desdits composés vectorisés d'une molécule analgésique dans une composition pharmaceutique pour la préparation d'un médicament utile pour le traitement de la douleur.

De préférence, la composition pharmaceutique se présente sous une forme appropriée pour une administration par voie systémique, par voie parentérale, par voie orale, par voie rectale, par voie nasale, par voie transdermique, par voie pulmonaire.

L'invention a également pour objet une méthode de traitement de la douleur consistant à administrer à un patient une composition pharmaceutique comprenant au moins un composé vectorisé constitué d'une molécule analgésique liée à un vecteur, ledit peptide étant un dérivé de la famille de la Protégrine ou de la Tachyplésine.

Les figures présentées en annexe illustrent différents résultats obtenus par la Demanderesse à la suite des travaux expérimentaux qui lui ont permis d'obtenir les composés vectorisés de l'invention:

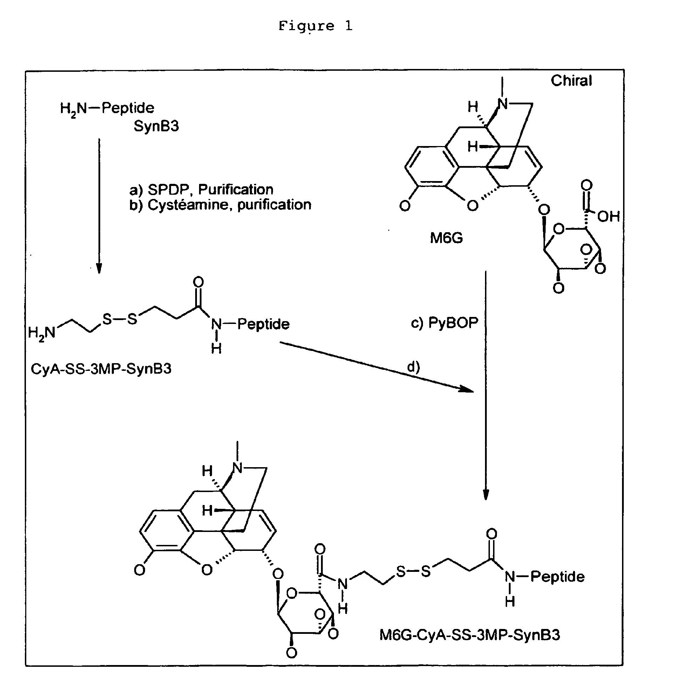

- la figure 1 représente schématiquement la synthèse chimique d'un composé vectorisé du morphine-6-glucuronide (M6G), métabolite de la morphine.
- la figure 2 illustre les résultats d'une étude comparative de l'efficacité analgésique entre le composé 1 (morphine) et le composé 2 (M6G vectorisée).
- la figure 3 illustre les résultats d'une étude comparative de l'efficacité analgésique entre le composé 1 (morphine), le composé 2 (M6G vectorisée) et le composé 3 (M6G libre).
- la figure 4 illustre une étude comparative de la pénétration dans la BHE de la M6G libre (composé 1) avec celle de la M6G vectorisée (composé 2).
- la figure 5 illustre les résultats d'une étude comparative de l'effet de la morphine, de la M6G vectorisée et de la M6G libre sur la dépression respiratoire. Les composés ont été utilisés à la dose ED50 (Figure 5, A), à la dose 5x ED50 (Figure 5, B) et à la dose 10x ED50 (Figure 5, C).

La présente invention sera mieux comprise à la lecture de la description des travaux expérimentaux effectués dans le cadre des recherches menées par la Demanderesse.

### I. Préparation des composés à tester.

### I.1. Synthèse Chimique de la M6G vectorisée.

### a) Synthèse du peptide vecteur.

Le peptide SynB3 est assemblé sur phase solide selon une stratégie Foc/tu, clivé et déprotégé par l'acide trifluoroacétique, puis purifié par chromatographie haute pression préparative en phase inverse et lyophilisé. Sa pureté (>95%) et son identité sont confirmées par HPLC analytique et par spectrométrie de masse.

### b) Couplage du maillon CyA-3MP sur le peptide vecteur.

Le peptide SynB3 de séquence RRLSYSRRRF (SEQ ID N° 1 dans la liste de séquence en annexe) (un équivalent molaire) est incubé pendant 30 minutes avec un équivalent molaire du réactif SPDP (S-pyridinium succinimidyl-mercaptopropionate) dans le solvant DMF (Diméthylformamide) en présence de deux équivalents molaires de DIEA (Diisopropyléthylamine). Le peptide résultant, Py-SS-3MP-SynB3 est précipité à l'éther, puis purifié par chromatographie haute pression préparative en phase inverse et lyophilisé. Sa pureté (>95%) et son identité sont confirmées par HPLC analytique et par spectrométrie de masse.

Un équivalent molaire de peptide Py-SS-3MP-SynB3 est incubé pendant 30 minutes avec quatre équivalents molaires de CyA, HC1 (Chlorhydrate de cystéamine) dans le solvant DMF (Diméthylformamide) en présence de quatre équivalents molaires de DIEA. Le peptide résultant, CyA-SS-3MP-SynB3 est précipité à l'éther, puis purifié par chromatographie haute pression préparative en phase inverse et lyophilisé. Sa pureté (>95%) et son identité sont confirmées par HPLC analytique et par spectrométrie de masse.

### c) Couplage de M6G sur CyA-SS-3MP-SynB3.

Un équivalent molaire de M6G est incubé avec deux équivalents molaires de PyBOP, en présence de quatre équivalents molaires de DIEA dans le solvant DMF.

Un équivalent molaire de peptide CyA-SS-3MP-SynB3 dissous dans DMF est ensuite ajouté au mélange réactionnel, puis incubé pendant 30 minutes. Le produit formé M6G-CyA-SS-3MP-SynB3 est précipité à l'éther, puis purifié par chromatographie haute pression préparative en phase inverse et lyophilisé. Sa pureté (>95%) et son identité sont confirmées par HPLC analytique et par spectrométrie de masse.

### 1.2. Les composés à tester.

Le tableau 1 ci-dessous récapitule les différents composés testés.

**Tableau 1**

| Composé | |
|---|---|
| Composé 1 | M6G |
| Composé 2 | M6G-S-S-RRLSYSRRRF |
| Composé 3 | Morphine |

### II. Comparaison de l'effet analgésique.

### II.1. Essai utilisé : test de la plaque chauffante.

### a) Conditions expérimentales.

Le test de la plaque chauffante est réalisé en suivant le protocole expérimental décrit par Eddy NB et al. Synthetic analgesics. 1 - Methadone isomers and derivatives. J. Pharmacol. Exp. Ther.1950 (98) :121-137.

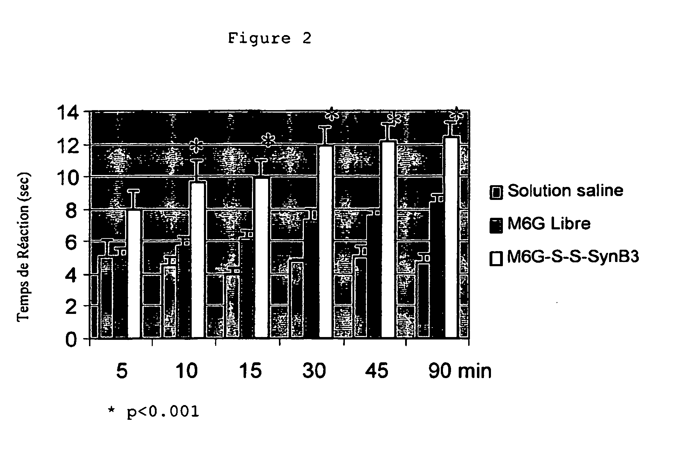

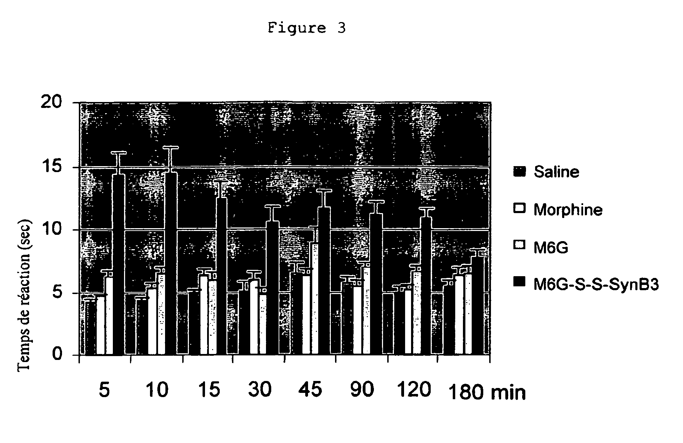

La souris déposée sur une plaque chauffée à 55°C manifeste sa douleur par le léchage des pattes antérieures, ou plus rarement par un saut. Le temps de réaction est alors noté. Les composés étudiés sont administrés par voie intraveineuse au niveau de la veine caudale de la souris à une dose de 1mg/kg. Le temps de réaction est alors mesuré après 5 à 90 minutes (résultats présentés en figure 2) et de 5 à 180 minutes (résultats présentés en figure 3).

### b) Résultats.

Dans un premier temps, les inventeurs ont comparé l'activité analgésique du composé M6G libre par rapport à celle du composé obtenu par vectorisation de la M6G avec le peptide SynB3. Les résultats obtenus sont représentés à la figure 2 et montrent clairement que l'effet analgésique de la M6G vectorisée (composé 2) est beaucoup plus significatif que celui obtenu avec la M6G libre (composé 1). Cet effet analgésique est lent et dure même après 90 minutes post-administration.

Dans une autre expérience, l'effet de la M6G vectorisée (composé 2) a été comparé à celui de la morphine (composé 3) à la même dose de 1 mg/Kg. Les résultats de cette expérience (Figure 3) montrent clairement que la M6G vectorisée (composé 2) a un effet analgésique beaucoup plus important que celui de la morphine et ceci à un temps allant jusqu'à 120 minutes post-administration.

### 11.2. Essai utilisé : Test de tail flick.

### a) Conditions expérimentales.

La queue de la souris est placée devant une source d'infrarouge. La lumière est focalisée sur la surface ventrale de la queue de façon à produire une température de surface de 55°C. Dès que la souris bouge la queue, le temps de réaction est alors mesuré. Les composés étudiés sont administrés par voie sous-cutanée. Trois mesures sont faites avant administration du produit pour avoir un temps de base. Le pourcentage de souris analgésiées est ensuite représenté par le nombre de souris ayant un temps de réaction qui est au moins le double du temps de base divisé par le nombre de souris totales. La dose ED50 représente la concentration qui donne 50% de souris analgésiées.

### b) Résultats.

Dans un premier temps, nous avons comparé l'effet analgésique des composés morphine ou M6G libre par rapport à celui du composé obtenu par vectorisation de la M6G avec le peptide SynB3, ceci par deux voies d'administration : intraveineuse et sous-cutanée. Nous avons déterminé dans le modèle « Tail Flick », pour chaque produit, la ED50 qui représente la dose qui donne un effet analgésique dans 50% de souris. Le tableau 2 montre que la dose nécessaire pour induire un effet analgésique chez 50% de souris était beaucoup plus faible pour la M6G vectorisée (composé 2) que pour la morphine (composé 3) ou la M6G libre (composé 1). Ceci indique clairement que l'effet analgésique de la M6G vectorisée est beaucoup plus significatif que celui des autres produits testés.

**Tableau 2: Comparaison de l'activité analgésique**

| | Voie d'admin | Morphine (µmol/Kg) | M6G (µmol/Kg) | M6G vectorisée (µmol/Kg) |
|---|---|---|---|---|
| ED50 | Sous-cutanée | 11,6 | 6,6 | 1,7 |
| | Intraveineuse | 15,4 | >5,7 | 1,08 |

Dans un deuxième temps, nous avons mesuré la durée de l'effet analgésique chez ces souris. Le tableau 3 montre que non seulement la M6G vectorisée a un effet plus analgésique mais cet effet dure plus longtemps que celui de la M6G ou de la morphine.

**Tableau 3: Comparaison du temps de l'effet analgésique**

| | voie d'admin | Morphine | M6G | M6G vectorisée |
|---|---|---|---|---|
| Durée de | Sous-cutanée | 90 min | 150 min | 300 min |
| l'effet | Intraveineuse | 60 min | 90 min | 180 min |

### III. Comparaison de l'affinité aux récepteurs.

### a) Conditions expérimentales.

Les homogénats de tissu sont préparés à partir de cerveaux de veau (thalamus pour µ1 et µ2 et cortex frontal pour delta). [³H] [D-Ala²,D-Leu⁵]enkephalin (DADLE)(0.7 nM) est incubé avec 3 ml d'homogénat de tissu (15 mg de tissu par ml) en présence de 10 nM de [D-Pen²,D-Pen⁵]enkephalin (DPDPE) et de concentrations croissantes de M6G libre ou vectorisée. Dans ces conditions, une liaison spécifique aux sites de binding de µl est observée. Pour le récepteur µ2, [D-Ala²,MePhe⁴,Gly(ol)⁵]enkephalin (DAMGO) (1 nM) est incubé avec 3 ml d'homogénat de tissu en présence de 5 nM [D-Ser², Leu⁵]enkephalin-Thr⁶ (DSLET) et des concentrations croissantes de M6G libre ou vectorisée. Pour le récepteur delta, le tissu est incubé avec du [³H] [D-Pen²,D-Pen⁵]enkephalin en présence de M6G libre ou vectorisée. Le binding non spécifique est déterminé grâce à l'addition aux ligands marqués de 1 µM de levallorphan.

### b) Résultats.

La M6G est un métabolite actif de la morphine qui se lie au récepteurs µ avec une très haute affinité. Nous avons comparé l'affinité de la M6G libre (composé 1) à celle de la M6G vectorisée (composé 2) aux récepteurs µ1, µ2 et delta.

Les résultats montrent que le fait de rajouter un linker et un peptide vecteur (SynB3) à la M6G augmente son affinité d'environ 3 fois au récepteur µ1 et de 10 fois au récepteur µ2. Par contre, la liaison au récepteur delta est restée la même.

**Tableau 4 : Affinité aux récepteurs µ1 et µ2 (Ki exprimée en nM).**

| composé | µ1 | µ2 | delta |
|---|---|---|---|
| M6G (composé 1) | 2,67 | 5,82 | 23 |
| M6G-S-S-SynB3 (composé 2) | 0,99 | 0,60 | 19 |

### IV. Comparaison de la pénétration dans le cerveau.

### a) Conditions expérimentales : Perfusion Cérébrale in situ.

Des souris (20-25 g, Iffa-Credo ; l'Arbresle, France) sont anesthésiées. Après exposition de la carotide commune, l'artère carotide externe droite est liée au niveau de la bifurcation avec la carotide interne et la carotide commune est liée entre le coeur et le site d'implantation du cathéter (cathéter polyéthylène, ID :0.76). Celui-ci, préalablement rempli par une solution d'héparine (100 unités/ml) est inséré dans la carotide commune. Les souris sont perfusées avec le tampon de perfusion (128 mM NaCl, 24 mM NaHCO₃, 4,2 mM KCl, 2,4 mM NaH₂PO₄, 1,5 mM CaCl₂, 0,9 mM MgSO₄, et 9 mM D-glucose). Ce tampon est filtré puis bullé par un mélange contenant 95% O₂/ 5% CO₂ afin de maintenir le pH proche de 7,4 et d'alimenter le cerveau en oxygène au cours de la perfusion.

Les souris sont perfusées avec le tampon contenant la M6G libre (composé 1 ; activité spécifique 84 mCi/mg) ou la M6G vectorisée (composé 2 ; activité spécifique 14,3 mCi/mg). Juste avant le début de la perfusion, le coeur est arrêté par section des ventricules, ceci afin d'éviter au cours de la perfusion un reflux du perfusat. L'hémisphère droit est alors perfusé à une vitesse de 10 ml/min pendant 60 secondes après quoi la souris est décapitée. La quantité de radioactivité dans l'hémisphère droit est alors mesurée et l'indice de pénétration cérébrale (Kin) est calculé.

### b. Résultats.

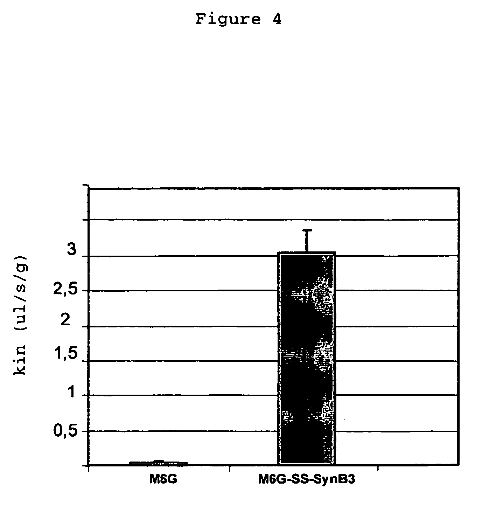

Dans cette étude, nous avons comparé la pénétration dans la BHE de la M6G libre (composé 1) avec celle de la M6G vectorisée (composé 2). Les deux produits ont été perfusés dans le cerveau de la souris. Après 60 secondes de perfusion dans le tampon, la pénétration des produits est estimée par la constante d'influx ou Kin en µl/sec/g. La figure 4 montre que la vectorisation de la M6G par le vecteur SynB3 augmente son passage dans le cerveau d'environ 100 fois après une perfusion de 60 secondes dans du tampon.

### V. Comparaison de la dépression respiratoire.

### a. Conditions expérimentales.

La dépression respiratoire a été étudiée chez le rat. Le produit est injecté aux animaux par voie sous-cutanée et après un certain temps, un aliquot de sang est prélevé de l'artère fémorale via un cathéter implanté préalablement. Durant l'étude, les animaux sont placés dans un endroit calme. Après le prélèvement sanguin, la saturation d'oxygène (SO₂) et le pression de CO₂ (PCO₂) sont mesurées. Les valeurs de SO₂ sont mesurées en % d'O₂.

Les rats ont été injectés avec 3 doses de chaque produit qui correspondent à ED50, 5xED50, 10xED50 (voir Tableau 5). Après des temps allant de 0 à 150 min (30, 60, 90, 120, 150 min), le sang est prélevé et la saturation en O₂ (SO₂) et la pression de CO₂ sont mesurées.

**Tableau 5 : Doses utilisées pour l'étude de dépression respiratoire.**

| | ED50 (µmol/kg) | 5xED50 (µmol/kg) | 10xED50 (µmol/kg) |
|---|---|---|---|
| Morphine | 13.1 | 66 | 131 |
| M6G | 8.7 | 43.7 | 87 |
| M6G vectorisée | 4.3 | 21.8 | 43 |

### b. Résultats.

Bien que la morphine soit la substance la plus utilisée dans le traitement de douleurs de moyenne et de grande intensité, son utilisation induit une dépression respiratoire chez les patients. Nous avons donc comparé l'effet de M6G vectorisée à celle de la morphine et de la M6G libre.

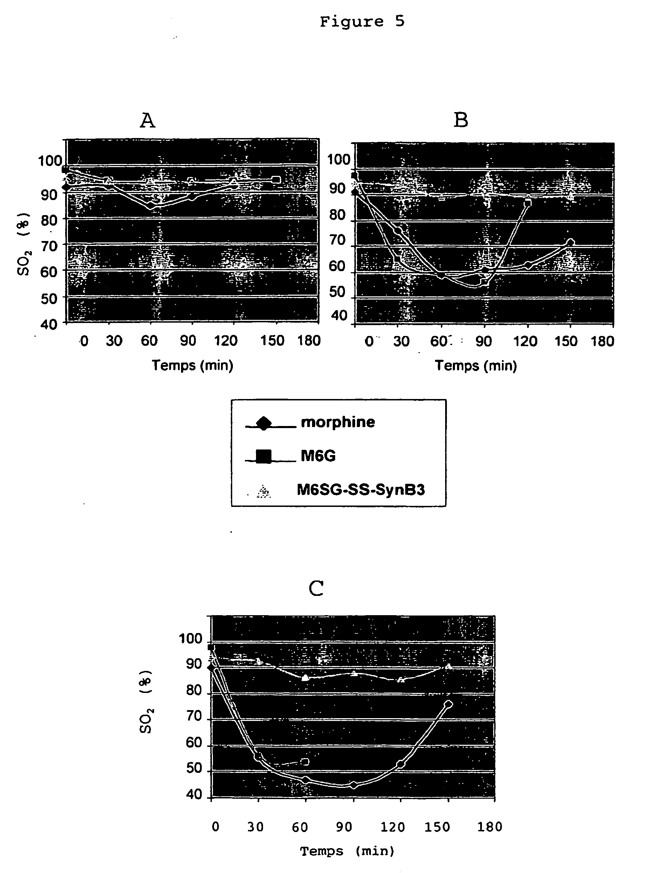

A la dose ED50 (Figure 5, A), aucun effet n'a été observé pour la M6G vectorisée ou la M6G. Nous avons noté, quand-même, une petite diminution entre 30 et 90 min de la saturation en O₂ pour la morphine.

Aux doses 5xED50 et 10xED50 (Figure 5, B et C), la morphine et la M6G ont induit une diminution très significative de la saturation d'oxygène. Le taux d'oxygène est descendu à presque 50% entre 30 et 90 min. Par contre, pour la M6G vectorisée, aucune diminution notable n'a été obtenue.

A la dose 10xED50 (Figure 5, C), la forte diminution observée avec la M6G libre a provoqué la mort de quelques animaux au temps 60 min.

Les résultats présentés à la figure 5 démontrent que le produit M6G vectorisée a non seulement un effet analgésique meilleur que la M6G libre ou la morphine mais permet également de diminuer de façon significative les effets secondaires associés avec la morphine.

## Revendications

1. Un composé **caractérisé en ce qu'**il est constitué d'une molécule analgésique qu'est la morphine-6-glucuronide (M6G) liée à un vecteur capable de transporter ladite molécule analgésique à travers la barrière hémato-encéphalique, ledit vecteur étant un peptide linéaire de séquence RRLSYSRRRF (SynB3, SEQ ID NO: 1 dans la liste de sequences).

2. Un composé selon la revendication 1, **caractérisé en ce que** les positions de liaison de la molécule analgésique sur le vecteur se situent au niveau des extrémités N-terminale ou C-terminale ou bien au niveau des chaînes latérales dudit vecteur.

3. Un composé selon l'une quelconque des revendications 1 ou 2, **caractérisé en ce que** la liaison entre la molécule analgésique et le vecteur est effectuée au moyen d'un groupe fonctionnel présent naturellement ou introduit soit sur le vecteur, soit sur la molécule analgésique, soit sur les deux.

4. Un composé selon la revendication 3, **caractérisé en ce que** le groupe fonctionnel est choisi parmi les groupes : -OH, -SH, -COOH, ou -NH₂.

5. Un composé selon l'une quelconque des revendications 1 à 4, **caractérisé en ce que** la liaison entre la molécule analgésique et le vecteur est une liaison choisie parmi une liaison covalente, une liaison hydrophobe, une liaison ionique, une liaison clivable ou une liaison non clivable dans les milieux physiologiques ou à l'intérieur des cellules.

6. Un composé selon l'une quelconque des revendications 1 à 5, **caractérisé en ce que** la liaison entre la molécule analgésique et le vecteur est une liaison directe.

7. Un composé selon l'une quelconque des revendications 1 à 5, **caractérisé en ce que** la liaison entre la molécule analgésique et le vecteur est une liaison indirecte mise en oeuvre au moyen d'un agent de liaison.

8. Un composé selon la revendication 7, **caractérisé en ce que** l'agent de liaison est choisi parmi des agents bi ou multifonctionnels contenant des groupements alkyle, aryle, aralkyle ou peptidique, des esters, aldéhydes ou acides d'alkyle, aryle ou aralkyle, des groupements anhydrides, sulfhydriles, ou carboxyles tels que les dérivés de l'acide maleymil benzoïque, de l'acide maleymil propionique et des dérivés succynimidyle, des groupes dérivés du bromure ou chlorure de cyanogène, carbonyldiimidazole, des esters de succinimide ou des halogénures sulphoniques.

9. Un composé selon l'une quelconque des revendications 1 à 8 **caractérisé en ce que** la liaison entre la molécule analgésique et le vecteur comporte au moins un pont disulfure.

10. Un composé selon la revendication 1, **caractérisé en ce que** le vecteur est fixé au niveau de l'acide carboxylique du résidu glucuronide de ladite molécule de morphine-6-glucuronide.

11. Utilisation d'un composé selon l'une quelconque des revendications 1 à 10 dans une composition pharmaceutique pour la préparation d'un médicament utile pour le traitement de la douleur.

12. Utilisation selon la revendication 11, **caractérisée en ce que** la composition pharmaceutique se présente sous une forme appropriée pour une administration par voie systémique, par voie parentérale, par voie orale, par voie rectale, par voie nasale, par voie transdermique, par voie pulmonaire.

## Claims

1. A compound **characterized in that** it is formed of an analgesic molecule which is the morphine-6-glucuronide (M6G) linked to a vector able to carry said analgesic molecule through the blood-brain barrier, the said vector being a linear peptide of sequence RRLSYSRRRF (SynB3, SEQ ID N:1 in the list of sequences).

2. Compound according to claim 1, **characterized in that** the binding positions of the analgesic molecule to the vector are located at the N-terminal or C-terminal ends, or at the side chains of said vector.

3. Compound according to any of claim 1 or 2, **characterized in that** the binding between the analgesic molecule and the vector is made by means of a functional group that is present naturally or inserted either in the vector or the analgesic molecule, or in both.

4. Compound according to claim 3, **characterized in that** the functional group is chosen from among the groups: -OH, -SH, -COOH or -NH₂.

5. Compound according to any of claims 1 to 4, **characterized in that** the bond between the analgesic molecule and the vector is a bond chosen from among a covalent bond, a hydrophobic bond, an ionic bond, a bond that is cleavable or non-cleavable in physiological media or inside cells.

6. Compound according to any of claims 1 to 5, **characterized in that** the bond between the analgesic molecule and the vector is a direct bond.

7. Compound according to any of claims 1 to 5, **characterized in that** the bond between the analgesic molecule and the vector is an indirect bond applied by means of a binding agent.

8. Compound according to claim 7, **characterized in that** the binding agent is chosen from among bi-or multifunctional agents containing alkyl, aryl, aralkyl or peptide groups, esters, alkyl, aryl or aralkyl aldehydes or acids, anhydride, sulfhydryl or carboxyl groups such as the derivatives of benzoic maleymil acid, propionic maleymil acid, and succinimidyl derivatives, derivative groups of cyanogen bromide or chloride, carbonyldiimidazole, esters of succinimide or sulfonic halides.

9. Compound according to any of claims 1 to 8, **characterized in that** the bond between the analgesic molecule and the vector contains at least one disulfide bridge.

10. Compound according to claim 1, **characterized in that** the vector is bound at the carboxyl group of the glucuronide residue of the said molecule of morphine-6-glucuronide.

11. Use of a compound according to any of claims 1 to 10 in a pharmaceutical composition for the preparation of a medicinal product useful for the treatment of pain.

12. Use according to claim 11, **characterized in that** the pharmaceutical composition is in an appropriate form for administration by parenteral route, oral route, rectal route, nasal route, transdermal route, pulmonary route.

## Patentansprüche

1. Eine Verbindung, **dadurch gekennzeichnet, dass** sie aus einem analgetischen Molekül, welches das Morphin-6-glucuronid (M6G) ist, gebunden mit einem Vektor, der in der Lage ist, das besagte analgetische Molekül durch die Blut-Hirn-Schranke zu transportieren, besteht, wobei der besagte Vektor ein lineares Peptid mit der Sequenz RRLSYSRRRF (SynB3, SEQ ID. Nr. 1 in der Sequenzliste) ist.

2. Eine Verbindung nach Anspruch 1, **dadurch gekennzeichnet, dass** sich die Bindungspositionen des analgetischen Moleküls auf dem Vektor auf Ebene des N-terminalen oder C-terminalen Endes oder auf Ebene der Seitenketten des besagten Vektors befinden.

3. Eine Verbindung nach einem der Ansprüche 1 oder 2, **dadurch gekennzeichnet, dass** die Bindung zwischen dem analgetischen Molekül und dem Vektor mittels einer funktionellen Gruppe durchgeführt wird, die natürlich vorhanden ist oder entweder in den Vektor oder in das analgetische Molekül oder in beide eingeführt wird.

4. Eine Verbindung nach Anspruch 3, **dadurch gekennzeichnet, dass** die funktionelle Gruppe ausgewählt ist aus den Gruppen -OH, -SH, -COOH oder -NH₂.

5. Eine Verbindung nach einem der Ansprüche 1 bis 4, **dadurch gekennzeichnet, dass** die Bindung zwischen dem analgetischen Molekül und dem Vektor eine Bindung ist, die ausgewählt ist aus einer kovalenten Bindung, einer hydrophoben Bindung, einer Ionenbindung, einer spaltbaren Bindung oder einer nicht spaltbaren Bindung in physiologischen Medien oder im Zellinnern.

6. Eine Verbindung nach einem der Ansprüche 1 bis 5, **dadurch gekennzeichnet, dass** die Bindung zwischen dem analgetischen Molekül und dem Vektor eine direkte Bindung ist.

7. Eine Verbindung nach einem der Ansprüche 1 bis 5, **dadurch gekennzeichnet, dass** die Bindung zwischen dem analgetischen Molekül und dem Vektor eine indirekte Bindung ist, die mit Hilfe eines Bindemittels durchgeführt wird.

8. Eine Verbindung nach Anspruch 7, **dadurch gekennzeichnet, dass** das Bindemittel ausgewählt ist aus den bi- oder multifunktionellen Mitteln, die Alkyl-, Aryl-, Aralkyl- oder Peptidgruppen enthalten, Ester, 20Aldehyde oder Alkyl-, Aryl- oder Aralkylsäuren, Anhydrid-, Sulfhydril- oder Carboxylgruppen wie die Derivate der Maleymilbenzoesäure, der Maleymilpropionsäure und Succinimidylderivate, vom Cyanogenbromid oder -chlorid abgeleitete Gruppen, Carbonyldiimidazol, Succinimidester oder Sulfon-Halogenide.

9. Eine Verbindung nach einem der Ansprüche 1 bis 8, **dadurch gekennzeichnet, dass** die Bindung zwischen dem analgetischen Molekül und dem Vektor mindestens eine Disulfidbrücke umfasst.

10. Eine Verbindung nach Anspruch 1, **dadurch gekennzeichnet, dass** der Vektor auf Ebene der Carboxylsäure des Glucuronidrests des besagten Morphin-6-glucuronid-Moleküls fixiert ist.

11. Verwendung einer Verbindung nach einem der Ansprüche 1 bis 10 in einer pharmazeutischen Zusammensetzung für die Zubereitung eines Medikaments zur Schmerzbehandlung.

12. Verwendung nach Anspruch 11, **dadurch gekennzeichnet, dass** sich die pharmazeutische Zusammensetzung in einer Form präsentiert, die für eine Verabreichung auf systemischem, parenteralem, oralem, rektalem, nasalem, transkutanem, pulmonalem Weg geeignet ist.
